# NEUE EUROPÄISCHE PATENTSCHRIFT

(11) **EP 2 027 881 B2**
(45) Veröffentlichungstag und Bekanntmachung des Hinweises auf die Entscheidung über den Einspruch: **12.07.2017**
(45) Hinweis auf die Patenterteilung: 26.10.2011
(21) Anmeldenummer: 08020454.8
(22) Anmeldetag: 18.06.2004
(51) Int. Cl.: A61M 25/00

(54) **Blasenkatheter-Set**
Bladder catheter set
Ensemble cathéter de vessie

(30) Priorität: 27.06.2003 DE 10329128
(43) Veröffentlichungstag der Anmeldung: 25.02.2009
(62) Teilanmeldung aus: 04738721.2
(73) Patentinhaber: Willy Rüsch GmbH, 71394 Kernen-Rommelshausen (DE)
(72) Erfinder: McBride, Barry, BT51 5EG Northern Ireland (GB)
(74) Vertreter: Grünecker Patent- und Rechtsanwälte PartG mbB

(56) Entgegenhaltungen:
- EP-A- 0 677 299
- EP-B- 0 923 398
- EP-B- 0 959 930
- WO-A-00/30575
- WO-A-97/41811
- WO-A1-01/43807
- WO-A1-98/06642
- WO-A1-98/11932
- WO-A2-03/002178
- DE-A- 2 317 839
- DE-A1- 3 223 535
- GB-A- 322 426
- GB-A- 2 319 507
- US-A- 3 154 080
- US-A- 4 140 127
- US-A- 4 140 127
- US-A- 4 811 847
- US-A- 5 226 530
- US-A- 5 454 798
- US-A- 6 090 075
- US-B1- 6 578 709

## Beschreibung

Die Erfindung betrifft ein Blasenkatheter-Set mit einer Verpackung eines, insbesondere intermediären, Blasenkatheters zum Einführen in die Harnröhre (Urethra). Die Verpackung umhüllt dabei den Blasenkatheter steril. Derartige Verpackungen sind vielfach bekannt, weil medizinische Instrumente steril verpackt ihrem Anwendungsbereich zugeführt werden müssen. Die Verpackung wird zerstört, wenn das medizinische instrument benötigt wird. Die Verpackung gewährleistet, dass das darin gelagerte Instrument bis zu dessen Einsatz steril bleibt.

Handelt es sich bei dem medizinischen Instrument um einen Katheter, so ist es üblich, dass der Katheter bei Gebrauch aus der Verpackung herausgenommen wird, indem man die Verpackung an einem Ende öffnet. Ein intermediärer, d. h. ein Kurzzeit-Blasenkatheter wird sodann an einem Ende mit einer Hand ergriffen und aus der Verpackung herausgezogen.

Beim Katherismus ist es zur Reduzierung der Gleitreibung bekannt, dass die Außenoberfläche des Katheters mit einer Flüssigkeit, z. B. einem Gleitmittel oder einer wässrigen Kochsalzlösung benetzt wird, damit der Katheter möglichst schmerzfrei und ohne weitere Irritationen hervorzurufen in ein Hohlorgan des menschlichen Körpers eingeführt werden kann. Es ist auch bekannt, die Außenoberfläche eines medizinischen Instruments mit medizinischen Wirkstoffen zu benetzen, damit diese Wirkstoffe im Hohlorgan an das an das Instrument angrenzende Gewebe abgegeben werden.

Ein Blasenkatheter weist hierzu bevorzugt eine hydrophile Außenoberfläche über dessen Schaftlänge auf, welche vor dem Öffnen der Verpackung mittels einer Flüssigkeit, die innerhalb eines in der Verpackung vorgesehenen Flüssigkeitsreservoirs bevorratet wird, aktiviert wird.

Die EP 0 959 930 B1 und die EP 0 923 398 B1 offenbaren ein Blasenkatheter-Set mit einer Verpackung für einen dort integrierten Blasenkatheter, in der auch ein Flüssigkeitsreservoir ausgebildet ist, das für die Aktivierung der hydrophilen Außenoberfläche des in der Verpackung gelagerten Katheters vorgesehen ist. Die Außenoberfläche dieses Blasenkatheters weist eine hydrophile Beschichtung auf. Zur Applikation des Blasenkatheters wird dieser nach Aktivieren der hydrophilen Oberfläche aus der Verpackung entnommen. Ein weiteres derartiges Blasenkatheter-Set ist aus der GB 2319507 A bekannt.

Eine weitere Möglichkeit zur Benetzung von Kathetern nennt die DE 2317839. Darin wird ein Katheter in einer Verpackung gelagert, die in zwei Bereiche unterteilt ist. Ein Bereich ist mit Benetzungsmittel gefüllt und nimmt das einführbare Ende des Katheters auf. Der andere Bereich beherbergt das trichterförmige andere Ende des Katheters. Beide Bereiche sind durch eine Einschnürung voneinander unterteilt, was eine Ausbreitung des Benetzungsmittels in den anderen Bereich verhindert. Alternativ kann am einführbaren Ende des Katheters auch ein Beutel mit Benetzungsmittel angeordnet sein, welcher sich durch Druck öffnet und das Benetzungsmittel freigibt.

Schließlich ist aus der WO 97/41811 ein Katheter bekannt, der in einem Auffangbeutel untergebracht ist. An der Entnahmeöffnung für den Katheter ist ein Reservoir mit Benetzungsmittel angeordnet, so dass der Katheter bei Entnahme ausreichend mit Benetzungsmittel in Kontakt kommt. Zur Lagerung der Katheteranordnung kann diese in einen weiteren Folienbeutel eingeschweißt werden.

Nachteilig bei den Blasenkatheter-Sets gemäß Stand der Technik ist es, dass der Blasenkatheter aufgrund seiner bestimmungsgemäßen Länge nach dem Herausnehmen aus der Verpackung, besonders für behinderte Menschen, schwer handhabbar ist. Insbesondere besteht die Gefahr, dass die Außenoberfläche des Katheters durch ein Festhalten mit einer Hand mit Mikroorganismen kontaminiert werden kann. Dies kann zu Entzündungen der Urethra und der Blase führen, was nicht selten zu zumindest unangenehmen Komplikationen führt.

Aus der US 4140127 ist ein Blasenkatheter-Set bekannt, das eine Hülse mit zwei gegenüberliegenden Seitenwänden und einer Manschette umfasst. Die Manschette ist von dem das vordere Ende des Katheters aufnehmenden Ende der Hülse aus zurückgefaltet und dient zur Aufnahme der Finger der den Katheter applizierenden Person.

Der Erfindung liegt die Aufgabe zugrunde, ein Blasenkatheter-Set bereitzustellen, welches die Nachteile des Standes der Technik vermeidet und insbesondere einfach, schnell und kostengünstig anzuwenden ist.

Diese Aufgabe wird durch die Vorrichtung des unabhängigen Anspruchs gelöst. Die abhängigen Ansprüche stellen bevorzugte Ausführungsformen der Erfindung dar.

Erfindungsgemäß wird die Aufgabe durch ein Blasenkatheter-Set gemäß Anspruch 1 gelöst. Das Katheter-Set zur Kurzzeitkatheterisierung umfasst eine sterile Verpackung besteht, in die der vorgesehene Blasenkatheter zusammen mit einer Manschette, bevorzugt eingeschweißt ist. Der Blasenkatheter weist einen Katheterschaft, eine Katheterspitze und ein auffangbehältnisseitiges, bevorzugt trichterförmiges, Ende auf. Die Manschette umfasst den Katheterschaft zumindest abschnittsweise. Die Manschette ist entlang des Katheterschafts verschiebbar angeordnet. Dies hat den Vorteil, dass erfindungsgemäß der Blasenkatheter mit der verschiebbaren Manschette benutzerfreundlich platziert werden kann. Dadurch kann der Katheter nach dessen Herausziehen aus der Verpackung ohne direktes Ergreifen der Katheteroberfläche, d. h. durch Ergreifen der Manschette, gehandhabt werden. Es wird eine aseptische Applikationstechnik des Katheters ermöglicht. Die Manschette hat den Vorteil, dass sterile Abschnitte des Blasenkatheters durch eine unsachgemäße Berührung nicht unzulässig infiziert werden. Zusätzliche Verpakkungsmaßnahmen können entfallen. Der Blasenkatheter weist eine hydrophile Oberfläche auf. Innerhalb der Verpackung ist ein Flüssigkeitsreservoir vorhanden, wobei die hydrophile Oberfläche mittels einer in dem Flüssigkeitsreservoir bevorrateten Flüssigkeit aktivierbar ist. Die aktivierte hydrophile Oberfläche dient zur Reduzierung der Gleitreibung bei der Applikation des Blasenkatheters. Mittels des Flüssigkeitsreservoirs kann die Aktivierung sehr einfach und anwenderfreundlich durch geführt werden, ohne dass zu dem Blasenkatheter-Set weiteres Zubehör benötigt wird. Die Manschette ist derart ausgebildet, dass sich die Flüssigkeit beim Aktivieren der hydrophilen Oberfläche des Blasenkatheters zumindest teilweise in der Manschette sammelt. Dabei bildet die Manschette eine Art Trichter und umfasst den Katheterschaft derart, dass die Oberfläche des Katheterschafts beim Verschieben der Manschette mit der Flüssigkeit weitgehend gleichmäßig benetzt wird.

Die Manschette ist bevorzugt als Kunststoffbeutel ausgeführt, welcher bevorzugt eine Länge von 10 bis 15 cm aufweist. Diese Länge ist optimal zum Greifen der Manschette mit einer Hand, da sie der Größe einer Hand entspricht. Die Ausführung als Kunststoffbeutel hat den Vorteil von geringen Herstellungskosten und geringem Volumen, wodurch die Aufnahme der Manschette innerhalb der Verpackung erleichtert wird.

Das Flüssigkeitsreservoir weist bevorzugt eine Sollbruchstelle auf, mittels der das Flüssigkeitsreservoir bei geschlossener Verpackung, bevorzugt durch Drükken auf das Flüssigkeitsreservoir, zerstörbar ist. Dabei benetzt die Flüssigkeit nach einem Zerstören des Flüssigkeitsreservoirs die Oberfläche des Katheters. Dies hat den Vorteil, dass die Aktivierung der hydrophilen Oberfläche unmittelbar vor der Anwendung des Katheters vorgenommen werden kann.

Das Flüssigkeitsreservoir ist innerhalb der Verpackung bevorzugt in einem an die Katheterspitze angrenzenden Bereich positioniert, derart, dass ein Platzen der Verpackung beim Zerstören des Flüssigkeitsreservoirs vermieden wird. Dies kann dadurch geschehen, dass einige Zentimeter Abstand zwischen einem inneren Verpackungsende an der Katheterspitze und dem Flüssigkeitsreservoir eingehalten sind. Würde sich das Flüssigkeitsreservoir direkt an dem Verpackungsende befinden, so bestünde die Gefahr dass, insbesondere wenn das Flüssigkeitsreservoir durch Drücken zerstört wird, die unter Druck stehende Flüssigkeit die Verpackung zum Platzen bringen oder zumindest beschädigen würde.

Bei der Flüssigkeit handelt es sich bevorzugt um Wasser oder bevorzugt 0,9 gewichtsprozentige Kochsalzlösung oder Gleitgel. Diese Substanzen haben sich für den genannten Zweck bewährt.

Die Manschette kann z. B. die Kunststoffbeutefform aufweisen. Der Katheter verläuft durch den Kunststoffbeutel, der Kunststoffbeutel umfasst also den Katheterschaft, wobei der Kunststoffbeutel an einem Ende des Kunststoffbeutels den Katheterschaft enger umfasst als an einem anderen Ende. Der Kunststoffbeutel bildet also eine Art Trichter mit einer Einfüllöffnung und einem Abflussloch. Das enger umfassende Ende ist dabei das Abflussloch des Trichters, wobei das Abflussloch den Katheterschaft derart eng umfasst, dass die im Kunststoffbeutel gesammelte Flüssigkeit, bei einem Verschieben des Kunststoffbeutels in Richtung des auffangbehältnisseitigen Endes des Blasenkatheters, gerade so stark aus dem Kunststoffbeutel austreten kann, dass die Oberfläche des Blasenkatheters mit der Flüssigkeit benetzt wird. Die Flüssigkeit tritt also durch das Lumen des Abflussloches aus, wobei die Benetzung über das Volumen des Lumens vorgenommen wird. Dies hat den Vorteil, dass eine gleichmäßige Benetzung der Katheteroberfläche garantiert wird, so dass ein schmerzfreies Applizieren bestmöglich sichergestellt ist.

In der allgemein vorgesehenen Verwendung des Kathetersets ist eine Benetzung des Katheterschaftes zur vollkommenen Aktivierung der hydrophilen Oberfläche über die Manschette nicht notwendig, weil sich die zur Aktivierung der hydrophilen Oberfläche vorgesehene Flüssigkeit in der geschlossenen Verpackung gleichmäßig verteilen kann, wenn man das Flüssigkeitsreservoir zerstört. Die Manschette dient vorrangig zur verbesserten Handhabung des Katheters und zur Reduzierung des für die sichere Anwendung notwendigen Verpackungsmaterials.

In einer besonderen Ausführungsform des Blasenkatheter-Sets ist am auffangbehältnisseitigen Ende des Katheters ein Auffangbehältnis angeordnet. Dadurch wird eine Katheterisierung auch dann möglich, wenn keine Entsorgungsmöglichkeit der abfließenden Flüssigkeit in Reichweite ist.

Die Verpackung des erfindungsgemäßen Blasenkatheter-Sets ist bevorzugt aus einer Tiefziehfolienschicht und einer Papiermaterialschicht aufgebaut, wobei der Blasenkatheter zwischen der Tiefziehfolienschicht und der Papiermaterialschicht angeordnet ist. Hierbei handelt es sich um eine besonders kostengünstige und sichere sterile Verpackungsart. Die Papiermaterialschicht weist dabei eine Dicke auf, die eine ausreichende Stabilität der Verpackung sicherstellt. Das Papiermaterial ist mit einer Schicht versehen, welche die Sterilität des Katheters und insbesondere die Dichtheit des Katheter-Sets nach Aktivierung der hydrophilen Oberfläche des Katheters, also insbesondere nach Zerstören des Flüssigkeitsreservoirs, garantiert.

Die Verpackung des Blasenkatheter-Sets weist bevorzugt im Bereich des auffangbehältnisseitigen Endes des Katheters (Trichterbereich), wiederum bevorzugt auf einer Verlängerungslinie des Katheterschafts ein Aufhängeloch auf. Der Durchmesser des Aufhängelochs kann im Zentimeterbereich liegen, wodurch ein. Aufhängen des Katheter-Sets, z. B. an eine Türklinke, ermöglicht wird. Dies erleichtert die Handhabung des Katheter-Sets weiter.

Weitere Vorteile ergeben sich aus der Beschreibung und den beigefügten Zeichnungen. Die vorstehend genannten und die noch weiter aufgeführten Merkmale der Erfindung können jeweils einzeln oder in Kombination miteinander verwendet werden. Die erwähnten Ausführungsformen sind nicht als abschließende Aufzählung zu verstehen, sondern haben vielmehr beispielhaften Charakter.

Die Erfindung wird nachfolgend anhand von Ausführungsbeispielen unter Bezugnahme auf die Zeichnungen näher erläutert.

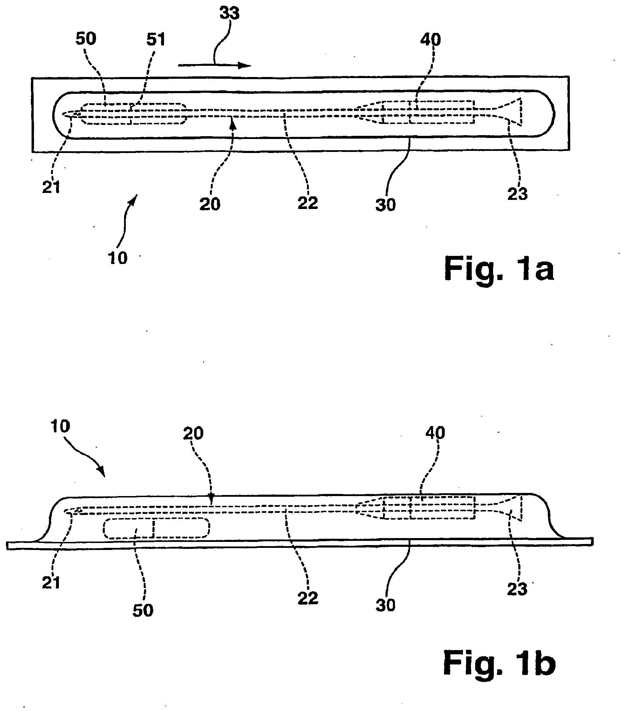

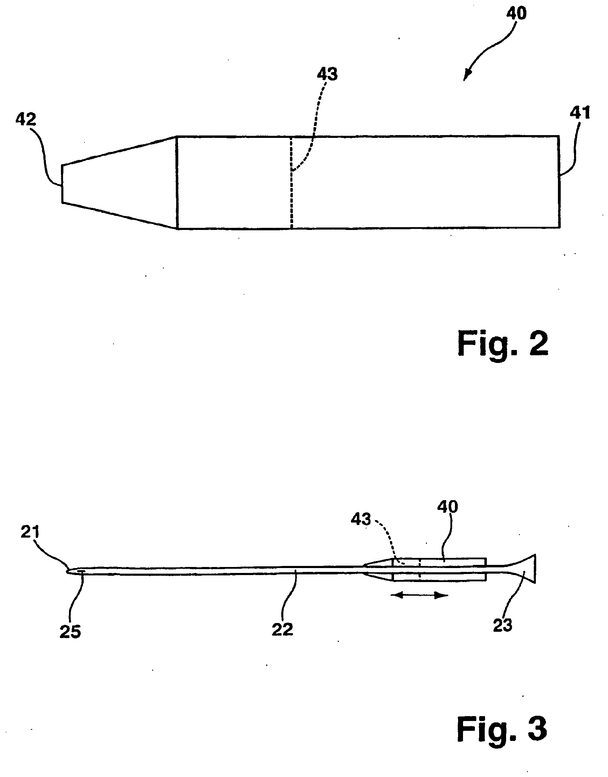

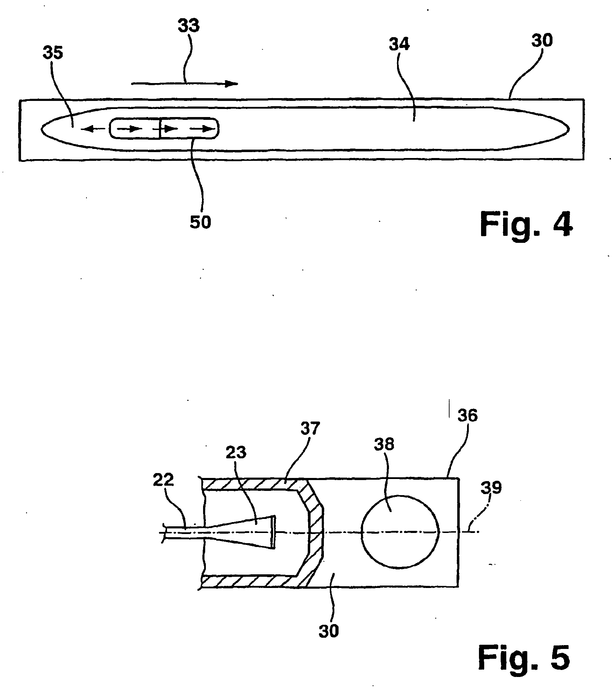

Fig. 1 mit den Fig. Ia und Ib zeigt ein erfindungsgemäßes Katheter-Set mit einem Blasenkatheter in dessen Verpackung in einer Draufsicht und einer Seitenansicht; Fig. 2 zeigt eine Ausführungsform der Manschette des erfindungsgemäßen Katheter-Sets ; Fig. 3 zeigt die Positionierung der Manschette auf dem Katheterschaft eines Katheters in einem erfindungsgemäßen Katheter-Set ; Fig. 4 zeigt eine bevorzugte Positionierung des Flüssigkeitsreservoirs eines erfindungsgemäßen Katheter-Sets ; und Fig. 5 im Ausschnitt zeigt eine bevorzugte Ausführungsform mit einem Aufhängeloch in der Verpackung eines erfindungsgemäßen Katheter-Sets.

Die Fig. der Zeichnungen zeigen den erfindungsgemäßen Gegenstand stark schematisiert und sind nicht maßstäblich zu verstehen. Die einzelnen Bestandteile des erfindungsgemäßen Gegenstandes sind so dargestellt, dass ihr Aufbau gut gezeigt werden kann.

In Fig. 1 wird ein erfindungsgemäßes Katheter-Set 10 mit einem Blasenkatheter 20 in dessen steriler Verpackung 30 dargestellt. Fig. Ia zeigt eine Draufsicht und Fig. Ib zeigt eine Seitenansicht des Katheter-Sets 10. Der Blasenkatheter 20 ist in seiner vollen Länge innerhalb der Verpackung platziert. Der Blasenkatheter 20 weist eine Katheterspitze 21, einen Katheterschaft 22 und ein trichterförmiges, auffangbehältnisseitiges Ende 23 auf. Innerhalb der Verpackung ist eine axial verschiebbare Manschette 40 angeordnet. Diese Manschette 40 umfasst den Blasenkatheter 20. Die Manschette 40 ist als flachgedrückter Folienschlauch ausgebildet. Weiter ist innerhalb der Verpackung 30 ein Flüssigkeitsreservoir 50 vorgesehen.

Der Blasenkatheter 20 weist eine hydrophile Außenoberfläche über die gesamte Länge seines Katheterschaftes 22 auf, und diese hydrophile Außenoberfläche ist über das Flüssigkeitsreservoir 50 aktivierbar, das ebenfalls in der Verpackung 30 integriert ausgebildet ist. Über eine Sollbruchstelle 51 kann man das Flüssigkeitsreservoir 50 in der Verpackung 30 zerstören. Dies geschieht z. B. durch Drücken auf das Flüssigkeitsreservoir 50 bis dieses platzt. Die innerhalb des Flüssigkeitsreservoirs 50 bevorratete Flüssigkeit breitet sich dann in vorbestimmten Bereichen der Verpackung 30 so aus, dass der darin gelagerte Blasenkatheter 20 an seiner Katheterschaftaußenoberfläche über die dafür vorgesehene Länge aktiviert wird.

In der Zeichnung ist bei horizontaler Lage der Verpackung die Ausbreitungsrichtung 33 der Flüssigkeit durch einen Pfeil angedeutet. Bevorzugt handelt es sich bei der Flüssigkeit um eine physiologische Kochsalzlösung (0,9 gewichtsprozentige Kochsalzlösung). Ist die Aktivierung der Außenoberfläche des Blasenkatheters 20 erfolgt, kann der Blasenkatheter 20 aus der Verpakkung derart genommen werden, dass man ihn mit einer Hand am auffangbehältnisseitigen Ende 23, das als Trichter ausgebildet ist, ergreift und den Blasenkatheter 20 der Länge nach aus der Verpackung 30 herauszieht. Längs des Katheterschaftes 22 ist die Manschette 40 ausgebildet, die beispielsweise mit der anderen Hand ergriffen werden kann. Die Manschette 40 ist bevorzugt entlang des Katheterschafts 22 verschiebbar ausgeführt und stellt eine innere Umhüllung des Katheters 20 zum Kontaminationsschutz des Teils des Katheters, der in die Harnröhre eingeführt werden soll dar. Diese Manschette 40 kann längs des Katheterschaftes 22 bis in den Spitzenbereich vorgeschoben werden.

Fig. 2 zeigt eine Ausführungsform der Manschette 40 des erfindungsgemäßen Katheter-Sets 10. Die Manschette 40 ist als Kunststoffbeutel ausgeführt, welcher die Form eines Trichters mit einer Einfüllöffnung 41 und einem Abflussloch 42 hat.. Der an die Einfüllöffnung 41 angrenzende Bereich ist zylinderförmig mit einer bevorzugten Länge von 10 cm. Die Einfüllöffnung 41 hat bevorzugt einen Durchmesser von 25 bis 30 mm. Der zylinderförmige Bereich geht über in einen konischen Abschnitt, mit einer bevorzugten Länge von 3 cm, welcher am Abflussloch 41 mit einem bevorzugten Durchmesser von 10 mm endet. Insgesamt hat die Manschette 40 damit eine Länge von 13 cm. Das Material der Manschette 40 ist bevorzugt sehr dünn und/oder oberflächenstrukturiert. Weiter kann die Manschette 40 z. B. im Abstand von 6 cm von dem Abflussloch 41 eine Perforation 43 zum leichten Auseinandertrennen der Manschette 40 aurweisen. Dadurch besteht die Möglichkeit, die Manschette 40 zu verkürzen, falls der Anwender dies als handlicher empfindet.

Fig. 3 zeigt die Positionierung der als Beutel ausgeführten Manschette 40, auf dem Katheterschaft 22 eines Katheters in einem erfindungsgemäßen Katheter-Set. Der Katheter weist z. B. eine Länge von 40 cm auf. Die Manschette 40 ist auf der Länge des Katheters, also entlang des Katheterschafts 22, verschiebbar. An der Katheterspitze 21 ist deren Öffnung 25, durch welche der Harn abfließen kann, eingezeichnet. Wird die Manschette 40 nach Aktivieren der hydrophilen Oberfläche des Katheters von der Katheterspitze 21 entlang des Katheterschafts 22 in Richtung des auffangbehältnisseitigen Endes 23 des Katheters verschoben, so führt dies zu einer gleichmäßigen Benetzung der Katheteroberfläche mit der zur Aktivierung verwendeten Flüssigkeit, da sich innerhalb der Manschette 40, d. h. im Beutel, zumindest Teile der Flüssigkeit befinden.

Fig. 4 zeigt eine bevorzugte Positionierung des Flüssigkeitsreservoirs eines erfindungsgemäßen Katheter-Sets in der Verpackung. Das Flüssigkeitsreservoir 50 platzt z. B. beim Ausüben einer Kraft von 20 Newton auf das Flüssigkeitsreservoir. Diese Kraft kann durch Drükken auf die Verpackung 30 erzeugt werden. Die Verpakkung 30 besteht bevorzugt aus einer Papiermaterialschicht und einer Tiefziehfolienschicht. Die Papiermaterialschicht kann dabei auch faserverstärkt sein. Der Blasenkatheter ist in üblicher verpackungstechnischer Weise zwischen diesen Schichten eingeschweißt, wobei der Blasenkatheter langgestreckt in einem von der Tiefziehfolienschicht gebildeten Hohlraum 34 angeordnet ist. Das Flüssigkeitsreservoir 50 ist in einem Abstand von ca. 4 plus/minus 1 cm vor dem katheterspitzenseitigen Ende 35 des Hohlraums 34, also in einem an die Katheterspitze angrenzenden Bereich, angeordnet. Der Abstand von ca. 4 cm zwischen Flüssigkeitsreservoir und dem Ende des Hohlraums wird eingehalten, damit das Risiko, dass der Hohlraum beim Zerstören, z. B. zum Platzen bringen des Flüssigkeitsreservoirs, aufreißt oder platzt, minimiert wird. Die Richtung einer Ausbreitung der im Flüssigkeitsbehälter bevorrateten Flüssigkeit innerhalb des Hohlraums beim Zerstören des Flüssigkeitsreservoirs 50 ist durch Pfeile eingezeichnet.

Fig. 5 zeigt eine bevorzugte Ausführungsform mit einem Aufhängeloch 38 in der Verpackung eines erfindungsgemäßen Katheter-Sets. Die Fig. zeigt den Bereich der Verpackung 30 des auffangbehältnisseitigen, trichterförmigen Endes 23 des Katheters. Das Aufhängeloch 38 ist in diesem Bereich angeordnet. Dazu ist die Verpackung in Richtung einer Verlängerungslinie 39 des Katheterschafts um ca. 4 cm, über einen Verschweißungsbereich 37, durch welchen die Papiermaterialschicht mit der Tiefziehfolienschicht verschweißt ist, verlängert. Das Aufhängeloch 38 weist z. B. einen Durchmesser von zwei Zentimetern und einen Abstand vom Verschweißungsbereich von einem Zentimeter auf. Das Aufhängeloch sollte mindestens 5 mm vom Verpakkungsrand 36 entfernt sein. Bezugszeichenliste 10 Katheter-Set 20 Blasenkatheter 21 Katheterspitze 22 Katheterschaft 23 auffangbehältnisseitiges Ende 25 Öffnung 30 Verpackung 33 Ausbreitungsrichtung 34 Hohlraum 35 katheterspitzenseitiges Ende 36 Verpackungsrand 37 Verschweißungsbereich 38 Aufhängeloch 39 Verlängerungslinie 40 Manschette 41 Einfüllöffnung 42 Abflussloch 43 Perforation 50 Flüssigkeitsreservoir 51 Sollbruchstelle

## Patentansprüche

1. Blasenkatheter-Set (10) mit einem in einer sterilen Verpackung (30) angeordneten Blasenkatheter (20), welcher einen Katheterschaft (22), eine Katheterspitze (21) und ein auffangbehältnisseitiges Ende (23) aufweist, wobei in der Verpackung (30) ein dünner Folienschlauch vorgesehen ist, der den Katheterschaft (22) abschnittsweise umfasst und entlang des Katheterschafts (22) verschiebbar angeordnet ist, der dünne Folienschlauch den Katheterschaft (22) an einem Ende des Folienschlauchs enger umfasst als an dem anderen Ende des Folienschlauchs, der dünne Folienschlauch kürzer ist als der Blasenkatheter (20), und der dünne Folienschlauch flachgedrückt ist.

2. Blasenkatheter-Set (10) nach Anspruch 1, **dadurch gekennzeichnet, dass** der Blasenkatheter (20) eine hydrophile Oberfläche aufweist und innerhalb der Verpackung (30) ein Flüssigkeitsreservoir (50) vorhanden ist, wobei die hydrophile Oberfläche mittels einer in dem Flüssigkeitsreservoir (50) bevorrateten Flüssigkeit aktivierbar ist.

3. Blasenkatheter-Set (10) nach Anspruch 2, **dadurch gekennzeichnet, dass dass** Flüssigkeitsreservoir (50) eine Sollbruchstelle (51) aufweist, mittels der das Flüssigkeitsreservoir (50) bei geschlossener Verpackung (30) zerstörbar ist, wobei die Flüssigkeit nach einem Zerstören des Flüssigkeitsreservoirs (50) die Oberfläche des Katheters (20) weitgehend vollständig benetzt.

4. Blasenkatheter-Set (10) nach Anspruch 2 oder 3, **dadurch gekennzeichnet, dass** das Flüssigkeitsreservoir (50) innerhalb der Verpackung (30) in einem an die Katheterspitze (21) angrenzenden Bereich positioniert ist.

5. Blasenkatheter-Set (10) nach mindestens einem der Ansprüche 2 bis 4, **dadurch gekennzeichnet, dass** die Flüssigkeit Wasser oder Kochsalzlösung oder Gleitgel ist.

6. Blasenkatheter-Set (10) nach Anspruch 5, **dadurch gekennzeichnet, dass** die Flüssigkeit 0,9 gewichtsprozentige Kochsalzlösung ist.

7. Blasenkatheter-Set (10) nach einem der Ansprüche 2 bis 6, **dadurch gekennzeichnet, dass** der dünne Folienschlauch derart ausgebildet ist, dass die Flüssigkeit beim Aktivieren der hydrophilen Oberfläche des Blasenkatheters (20) sich zumindest teilweise in dem dünnen Folienschlauch sammelt, wobei der dünne Folienschlauch derart den Katheterschaft (22) umfasst, dass die Oberfläche des Katheterschafts (22) beim Verschieben des dünnen Folienschlauchs mit der Flüssigkeit weitgehend gleichmäßig benetzbar ist.

8. Blasenkatheter-Set (10) nach mindestens einem der Ansprüche 1 bis 7, **dadurch gekennzeichnet, dass** am auffangbehältnisseitigen Ende (23) des Katheters (20) ein Auffangbehältnis angeordnet ist.

9. Blasenkatheter-Set (10) nach mindestens einem der Ansprüche 1 bis 8, **dadurch gekennzeichnet, dass** die Verpackung (30) aus einer Tiefziehfolienschicht und Papiermaterialschicht aufgebaut ist, wobei der Blasenkatheter (20) zwischen der Tiefziehfolienschicht und der Papiermaterialschicht angeordnet ist.

10. Blasenkatheter-Set (10) nach mindestens einem der Ansprüche 1 bis 9, **dadurch gekennzeichnet, dass** die Verpackung (30) im Bereich des auffangbehältnisseitigen Endes (23) des Katheters (20) ein Aufhängeloch (38) aufweist.

11. Blasenkatheter-Set (10) nach mindestens einem der Ansprüche 7 bis 10, **dadurch gekennzeichnet, dass** das enger umfassende Ende (23) des dünnen Folienschlauchs den Katheterschaft (22) derart eng umfasst, dass die im dünnen Folienschlauch gesammelte Flüssigkeit, bei einem Verschieben des dünnen Folienschlauchs in Richtung des auffangbehältnisseitigen Endes (23) des Blasenkatheters (20), gerade so stark aus dem Folienschlauch austreten kann, dass die Oberfläche des Blasenkatheters (20) mit der Flüssigkeit benetzbar ist.

## Claims

1. Urinary catheter set (10) having a urinary catheter (20) which is arranged in a sterile package (30) and has a catheter shaft (22), a catheter tip (21) and an end (23) on the collecting pouch side, wherein in the package (30) a thin plastic sheeting tube is provided, which encloses the catheter shaft (22) in at least a section and is arranged so that it can move along the catheter shaft (22), the thin plastic sheeting tube encloses the catheter shaft (22) more tightly at one end of the plastic sheeting tube than at the other end of the plastic sheeting tube, the thin plastic sheeting tube is shorter than the urinary catheter (20) and the thin plastic sheeting tube is pressed flat.

2. Urinary catheter set (10) according to Claim 1, **characterised in that** the urinary catheter (20) has a hydrophilic surface and there is a fluid reservoir (50) inside the package (30), wherein the hydrophilic surface can be activated by means of a fluid stored in the fluid reservoir (50).

3. Urinary catheter set (10) according to Claim 2, **characterised in that** the fluid reservoir (50) has a rupture point (51), by means of which the fluid reservoir (50) can be broken open when the package (30) is closed, wherein the fluid, after breaking open the fluid reservoir (50), by and large completely wets the surface of the catheter (20).

4. Urinary catheter set (10) according to Claim 2 or 3, **characterised in that** the fluid reservoir (50) is positioned inside the package (30) in an area adjacent to the catheter tip (21).

5. Urinary catheter set (10) according to at least one of Claims 2 to 4, **characterised in that** the fluid is water or saline solution or lubricating gel.

6. Urinary catheter set (10) according to Claim 5, **characterised in that** the fluid is 0.9 per cent by weight saline solution.

7. Urinary catheter set (10) according to any one of Claims 2 to 6, **characterised in that** the thin plastic sheeting tube is designed in such a way that the fluid, when the hydrophilic surface of the urinary catheter (20) is activated, at least partly collects in the thin plastic sheeting tube, wherein the thin plastic sheeting tube encloses the catheter shaft (22) in such a way that the surface of the catheter shaft (22) can by and large be uniformly wetted with the fluid when the thin plastic sheeting tube is moved.

8. Urinary catheter set (10) according to at least one of Claims 1 to 7, **characterised in that** a collecting pouch is arranged at the end (23) of the catheter (20) on the collecting pouch side.

9. Urinary catheter set (10) according to at least one of Claims 1 to 8, **characterised in that** the package (30) is constructed from a deep-drawn film layer and paper material layer, wherein the urinary catheter (20) is arranged between the deep-drawn film layer and the paper material layer.

10. Urinary catheter set (10) according to at least one of Claims 1 to 9, **characterised in that** the package (30), in the area of the end (23) of the catheter (20) on the collecting pouch side, has a suspension hole (38).

11. Urinary catheter set (10) according to at least one of Claims 7 to 10, **characterised in that** the more tightly enclosing end (23) of the thin plastic sheeting tube tightly encloses the catheter shaft (22) in such a way that when the thin plastic sheeting tube is moved in the direction of the end (23) of the urinary catheter (20) on the collecting pouch side, the fluid which has collected in the thin plastic sheeting tube can flow out from the plastic sheeting tube just sufficiently, so that the surface of the urinary catheter (20) is wetted with the fluid.

## Revendications

1. Ensemble cathéter de vessie (10) avec un cathéter de vessie (20) qui est disposé dans un emballage stérile (30) et qui comporte une tige de cathéter (22), une pointe de cathéter (21) et une extrémité située côté récipient collecteur (23), étant précisé qu'il est prévu dans l'emballage (30) un fin tube en film qui entoure par zones la tige (22) et qui est disposé de manière à pouvoir glisser le long de celle-ci, que le fin tube en film entoure la tige (22) plus étroitement à une extrémité qu'à l'autre, que le fin tube en film est plus court que le cathéter de vessie (20) et que le fin tube en film est aplati.

2. Ensemble cathéter de vessie (10) selon la revendication 1 **caractérisé en ce que** le cathéter de vessie présente une surface hydrophile et qu'il est prévu un réservoir de liquide (50) à intérieur de l'emballage (30), étant précisé que la surface hydrophile est apte à être activée à l'aide d'un liquide stocké dans le réservoir (50).

3. Ensemble cathéter de vessie (10) selon la revendication 2, **caractérisé en ce que** le réservoir de liquide (50) présente un point de rupture (51) à l'aide duquel ledit réservoir (50) peut être détruit alors que l'emballage (30) est fermé, étant précisé que le liquide, après une destruction du réservoir (50), humidifie quasi entièrement la surface du cathéter (20).

4. Ensemble cathéter de vessie (10) selon la revendication 2 ou 3, **caractérisé en ce que** le réservoir de liquide (50) est apte à être positionné à l'intérieur de l'emballage (30) dans une zone voisine de la pointe de cathéter (21).

5. Ensemble cathéter de vessie (10) selon l'une au moins des revendications 2 à 4, **caractérisé en ce que** le liquide est constitué par de l'eau ou une solution saline ou un gel lubrifiant.

6. Ensemble cathéter de vessie (10) selon la revendication 5, **caractérisé en ce que** le liquide est constitué par une solution saline à 0,9% en poids.

7. Ensemble cathéter de vessie (10) selon l'une des revendications 2 à 6, **caractérisé en ce que** le fin tube en film est conçu de telle sorte que le liquide, lors de l'activation de la surface hydrophile du cathéter de vessie (20), s'accumule en partie dans le fin tube en film, étant précisé que ce dernier entoure la tige de cathéter (22) de telle sorte que la surface de la tige (22) puisse être humidifiée quasi uniformément avec le liquide lors du glissement du fin tube en film.

8. Ensemble cathéter de vessie (10) selon l'une au moins des revendications 1 à 7, **caractérisé en ce qu'**il est prévu à l'extrémité située côté récipient collecteur (23) du cathéter (20) un récipient collecteur.

9. Ensemble cathéter de vessie (10) selon l'une au moins des revendications 1 à 8, **caractérisé en ce que** l'emballage (30) se compose d'une couche thermoformée et d'une couche de matériau en papier, étant précisé que le cathéter de vessie (20) est disposé entre la couche thermoformée et la couche de matériau en papier.

10. Ensemble cathéter de vessie (10) selon l'une au moins des revendications 1 à 9, **caractérisé en ce que** l'emballage (30) présente, dans la zone de l'extrémité située côté récipient collecteur (23) du cathéter (20), un trou de suspension (38).

11. Ensemble cathéter de vessie (10) selon l'une au moins des revendications 7 à 10, **caractérisé en ce que** l'extrémité plus serrée (23) du fin tube en film entoure la tige de cathéter (22) tellement étroitement que lors du glissement du tube en film en direction de l'extrémité située côté récipient collecteur (23) du cathéter de vessie (20), le liquide accumulé dans le fin tube en film puisse en sortir exactement avec la force nécessaire pour que la surface du cathéter (20) puisse être humidifiée avec le liquide.
